# EUROPÄISCHE PATENTSCHRIFT

(11) **EP 3 404 436 B1**
(45) Veröffentlichungstag und Bekanntmachung des Hinweises auf die Patenterteilung: **21.04.2021**
(21) Anmeldenummer: 17172018.8
(22) Anmeldetag: 19.05.2017
(51) Int. Cl.: G01R 33/48, G06T 7/00, A61B 5/055, G06K 9/20, A61B 5/00, G06K 9/62

(54) **FUNKTIONELLE MAGNETRESONANZ-BILDGEBUNG**
FUNCTIONAL MAGNETIC RESONANCE IMAGING
IMAGERIE PAR RÉSONANCE MAGNÉTIQUE FONCTIONNELLE

(43) Veröffentlichungstag der Anmeldung: 21.11.2018
(73) Patentinhaber: Siemens Healthcare GmbH, 91052 Erlangen (DE)
(72) Erfinder: Beck, Thomas, 91077 Dormitz (DE)

(56) Entgegenhaltungen:
- MADIHA J. JAFRI ET AL: "Functional Classification of Schizophrenia Using Feed Forward Neural Networks", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA., 3. September 2006 (2006-09-03), Seiten 6631-6634, XP055423279, Piscataway, NJ, USA DOI: 10.1109/IEMBS.2006.260906 ISBN: 978-1-4244-0032-4
- VANESSA SOCHAT ET AL: "A Robust Classifier to Distinguish Noise from fMRI Independent Components", PLOS ONE, Bd. 9, Nr. 4, 18. April 2014 (2014-04-18), Seite e95493, XP055423391, DOI: 10.1371/journal.pone.0095493
- YANLU WANG ET AL: "Dimensionality of ICA in resting-state fMRI investigated by feature optimized classification of independent components with SVM", FRONTIERS IN HUMAN NEUROSCIENCE, Bd. 9, 8. Mai 2015 (2015-05-08), XP055423392, DOI: 10.3389/fnhum.2015.00259
- M MCKEOWN: "Independent component analysis of functional MRI: what is signal and what is noise?", CURRENT OPINION IN NEUROBIOLOGY., Bd. 13, Nr. 5, Oktober 2003 (2003-10), Seiten 620-629, XP055423395, GB ISSN: 0959-4388, DOI: 10.1016/j.conb.2003.09.012

## Beschreibung

Verschiedene Beispiele der Erfindung betreffen Techniken, um Bilddaten der funktionellen Magnetresonanz-Bildgebung auszuwerten. Insbesondere betreffen verschiedene Beispiele Techniken, um Korrelationsmuster von neurophysikalische Ereignissen und Falschmuster im Rahmen einer Unabhängigkeitsanalyse zu trennen.

Die Magnetresonanz(MR)-Bildgebung (engl. magnetic-resonance imaging, MRI) kann dazu verwendet werden, um neurophysikalische Ereignisse zu analysieren. Insbesondere kann die MRI dazu verwendet werden, um in Bezug auf neurophysikalische Ereignisse funktionell korrelierte Regionen des Gehirns (neuronale Netzwerke) zu analysieren. Die neuronalen Netzwerke können durch Identifikation von Korrelationsmustern der neurophysikalischen Ereignisse sichtbar gemacht werden. Die Korrelationsmuster können dabei eine zeitlich und/oder räumliche Korrelation von neurophysikalischen Ereignissen bezeichnen.

Eine entsprechende Technik ist die funktionelle MRI (fMRI). Bei der fMRI werden zeitliche Änderungen eines Bildkontrastes mittels geeigneter bildgebender Messsequenzen der MRI dargestellt. Beispielsweise kann ein vom Blutsauerstoffgehalt abhängiger Kontrast im Rahmen der fMRI betrachtet werden, auch als BOLD-Kontrast bezeichnet (englisch blood oxygenation level dependent). Dadurch können neurophysikalische Ereignisse gemessen werden.

Eine spezielle Technik der fMRI ist die sogenannte Ruhezustand fMRI (englisch resting-state fMRI, rsfMRI). Bei der rsfMRI wird die Zeitabhängigkeit zwischen im Ortsraum beabstandeten neurophysikalischen Ereignissen (funktionale Konnektivität) berücksichtigt, wobei dem neuronalen Netzwerk keine oder keine signifikanten äußeren Reize vorgegeben werden. Beispielsweise wird bei der rsfMRI darauf verzichtet, die Untersuchungsperson bestimmte Tätigkeiten oder Gedanken ausführen zu lassen (Ruhezustand).

Bei der rsfMRI werden umfangreiche Zeitserien von dreidimensionalen Bilddaten analysiert, um die Funktionsweise und Korrelationen der Hirnaktivität im Ruhezustand zu untersuchen. Durch beschleunigte Bildgebungssequenzen kann eine besonders hohe Zeitauflösung erreicht werden. Beispielsweise können mehrere zweidimensionale Schichten in einem Untersuchungsbereich einer Untersuchungsperson gleichzeitig angeregt und ausgelesen werden. Siehe zum Beispiel Souza, S. P., et al. "SIMA: simultaneous multislice acquisition of MR images by Hadamard-encoded excitation." Journal of computer assisted tomography 12.6 (1988): 1026-1030; und Setsompop, Kawin, et al. "Blipped-controlled aliasing in parallel imaging for simultaneous multislice echo planar imaging with reduced g-factor penalty." Magnetic Resonance in Medicine 67.5 (2012): 1210-1224; und Breuer, Felix A., et al. "Controlled aliasing in parallel imaging results in higher acceleration (CAIPIRINHA) for multi-slice imaging." Magnetic resonance in medicine 53.3 (2005): 684-691.

Durch die höhere Zeitauflösung steigt die Menge an MR-Daten. Die zu analysierende Datenmenge erhöht sich, wodurch die rechenintensive Vorverarbeitung der Bilddaten deutlich mehr Zeit in Anspruch nimmt. Typische Zeitserien umfassen einen Zeitraum von ca. 6-8 Minuten und können beispielsweise 700-1000 Volumenbereiche abbilden, jeweils mit 70-80 Schichten.

Bei der rsfMRI werden unterschiedliche Techniken zur Auswertung der Zeitserien von Bilddaten verwendet. Eine Auswertung beruht auf einer Unabhängigkeitsanalyse (englisch independent component analysis, ICA). Die ICA beruht herkömmlicherweise auf Modellannahmen in Abhängigkeit des aktuellen Datensatzes, wie beispielsweise Saatpunkten, die a-priori die Auswertung steuern oder Beschreibungen eines externen Stimulus. Dies bedeutet, dass die Korrelationsmuster ohne eine a-priori Einschränkung erkannt werden können. Bei der ICA werden Korrelationsmuster in den zu Grunde liegenden Bilddaten gesucht, welche die Intesitätsänderungen der Zeitserie von Bilddaten als Folge neurofunktionaler Ereignisse erklären können. Dabei werden voneinander unabhängige Kandidaten-Korrelationsmuster durch die ICA aufgefunden. Diese werden auch als Komponenten der ICA bezeichnet. Die Vielzahl von Kandidaten-Korrelationsmuster umfasst dabei Korrelationsmuster der Vielzahl von neurophysikalischen Ereignissen, sowie Falschmuster, die z.B. aufgrund von Rauschen in den Bilddaten oder zwangsläufig aus dem verwendeten Algorithmus der ICA erhalten werden. Die Falschmuster sind oftmals von untergeordnetem Interesse und sollen aussortiert werden.

Bei herkömmlichen Techniken der rsfMRI kann es vergleichsweise aufwendig sein, eine Trennung zwischen den Korrelationsmustern der neurophysikalische Ereignisse einerseits und den Falschmustern andererseits herbeizuführen. Beispielsweise kann es erforderlich sein, eine große Anzahl von Kandidaten-Korrelationsmustern, die durch die ICA identifiziert werden, manuell als relevantes Korrelationsmuster der neurophysikalischen Ereignisse oder als Falschmuster zu klassifizieren. Dies kann zeitaufwendig und fehleranfällig sein.

Die Verwendung der ICA zur Identifikation von jenen Korrelationsmustern, denen neurophysikalische Ereignissen zugrunde liegen, wie auch die Anwendung eines computer-implementierten Klassifikators auf die identifizierten Korrelationsmuster ist bekannt aus Madiha J. Jafri et al: "Functional Classification of Schizophrenia Using Forward Neural Networks", Conference Proceedings. Annual International Conference of the IEEE Engineering in Medicine and Biology Society (IEEE Cat. No. 06CH37748); 30 Aug.-3 Sept. 2006; New York, NY, USA. 1. Januar 2006 (2006-01-01), Seiten 6631 - 6634. Der in der genannten Publikation erwähnte Klassifikator eignet sich jedoch nicht zur Trennung der Korrelationsmuster von Falschmustern. Diesbezüglich sei verwiesen auf Vanessa Sochat et al: "A Robust Classifier to Distinguish Noise from fMRI Independent Components", PloS ONE, Bd. 9, Nr. 4, 18. April 2014 (2014-04-18), Seite e95493.

Deshalb besteht ein Bedarf für verbesserte Techniken zur MRI von neurophysikalischen Ereignissen im Gehirn einer Untersuchungsperson. Insbesondere besteht ein Bedarf für rsfMRI Techniken, die zumindest einige der oben genannten Nachteile und Einschränkungen beheben oder lindern.

Diese Aufgabe wird durch die Merkmale der unabhängigen Patentansprüche gelöst. Die Merkmale der abhängigen Patentansprüche definieren Ausführungsformen.

Ein Verfahren gemäß Patentanspruch 1 zur MRI umfasst insbesondere das Erhalten einer Zeitserie von Bilddaten. Die Zeitserie von Bilddaten bildet eine Vielzahl von neurophysikalischen Ereignissen im Gehirn einer Untersuchungsperson ortsaufgelöst ab. Das Verfahren umfasst weiterhin das Durchführen einer ICA zur Identifikation einer Vielzahl von Kandidaten-Korrelationsmustern basierend auf der Zeitserie von Bilddaten. Die Vielzahl von Kandidaten-Korrelationsmustern umfasst dabei Korrelationsmuster der Vielzahl von neurophysikalischen Ereignisse sowie Falschmuster, z.B. aufgrund von Rauschen oder durch andere Effekte verursachte Intensitätsänderungen wie beispielsweise physiologische Effekte - z.B. Herzschlag oder Atmung - oder Erwärmung des Scanners. Das Verfahren umfasst weiterhin das Bestimmen eines Teilbereichs des Gehirns. Das Verfahren umfasst weiterhin für jedes Kandidaten-Korrelationsmuster aus der Vielzahl von Kandidaten-Korrelationsmustern: Bestimmen ein entsprechenden Werts einer vorgegebenen Metrik. Die Metrik indiziert eine Intensität des jeweiligen Kandidaten-Korrelationsmusters im Teilbereich. Das Verfahren umfasst weiterhin das Auswählen mindestens eines Kandidaten-Korrelationsmusters aus der Vielzahl von Kandidaten-Korrelationsmuster basierend auf den bestimmten Werten der vorgegebenen Metrik. Das Verfahren umfasst auch das Markieren des ausgewählten mindestens einen Kandidaten-Korrelationsmusters für eine Analyse durch einen Benutzer.

Ein Computerprogrammprodukt umfasst Programmcode, der von mindestens einem Prozessor ausgeführt werden kann. Das Ausführen des Programmcodes bewirkt, dass der mindestens eine Prozessor ein Verfahren durchführt. Das Verfahren umfasst das Erhalten einer Zeitserie von Bilddaten. Die Zeitserie von Bilddaten bildet eine Vielzahl von neurophysikalischen Ereignissen im Gehirn einer Untersuchungsperson mit überlagertem Rauschen ortsaufgelöst ab. Das Verfahren umfasst weiterhin das Durchführen einer ICA zur Identifikation einer Vielzahl von Kandidaten-Korrelationsmustern basierend auf der Zeitserie von Bilddaten. Die Vielzahl von Kandidaten-Korrelationsmustern umfasst dabei Korrelationsmuster der Vielzahl von neurophysikalischen Ereignisse sowie Falschmuster, z.B. aufgrund des Rauschen oder durch andere Effekte verursachte Intensitätsänderungen wie beispielsweise physiologische Effekte oder Erwärmung des Scanners. Das Verfahren umfasst weiterhin das Bestimmen eines Teilbereichs des Gehirns. Das Verfahren umfasst weiterhin für jedes Kandidaten-Korrelationsmuster aus der Vielzahl von Kandidaten-Korrelationsmustern: Bestimmen eines entsprechenden Werts einer vorgegebenen Metrik. Die Metrik indiziert eine Intensität des jeweiligen Kandidaten-Korrelationsmusters im Teilbereich. Das Verfahren umfasst weiterhin das Auswählen mindestens eines Kandidaten-Korrelationsmusters aus der Vielzahl von Kandidaten-Korrelationsmuster basierend auf den bestimmten Werten der vorgegebenen Metrik. Das Verfahren umfasst auch das Markieren des ausgewählten mindestens einen Kandidaten-Korrelationsmusters für eine Analyse durch einen Benutzer.

Ein Computerprogramm umfasst Programmcode, der von mindestens einem Prozessor ausgeführt werden kann. Das Ausführen des Programmcodes bewirkt, dass der mindestens eine Prozessor ein Verfahren durchführt. Das Verfahren umfasst das Erhalten einer Zeitserie von Bilddaten. Die Zeitserie von Bilddaten bildet eine Vielzahl von neurophysikalischen Ereignissen im Gehirn einer Untersuchungsperson mit überlagertem Rauschen ortsaufgelöst ab. Das Verfahren umfasst weiterhin das Durchführen einer ICA zur Identifikation einer Vielzahl von Kandidaten-Korrelationsmustern basierend auf der Zeitserie von Bilddaten. Die Vielzahl von Kandidaten-Korrelationsmustern umfasst dabei Korrelationsmuster der Vielzahl von neurophysikalischen Ereignissen sowie Falschmuster, z.B. aufgrund des Rauschen oder durch andere Effekte verursachte Intensitätsänderungen wie beispielsweise physiologische Effekte oder Erwärmung des Scanners. Das Verfahren umfasst weiterhin das Bestimmen eines Teilbereichs des Gehirns. Das Verfahren umfasst weiterhin für jedes Kandidaten-Korrelationsmuster aus der Vielzahl von Kandidaten-Korrelationsmustern: Bestimmen eines entsprechenden Werts einer vorgegebenen Metrik. Die Metrik indiziert eine Intensität des jeweiligen Kandidaten-Korrelationsmusters im Teilbereich. Das Verfahren umfasst weiterhin das Auswählen mindestens eines Kandidaten-Korrelationsmusters aus der Vielzahl von Kandidaten-Korrelationsmuster basierend auf den bestimmten Werten der vorgegebenen Metrik. Das Verfahren umfasst auch das Markieren des ausgewählten mindestens einen Kandidaten-Korrelationsmusters für eine Analyse durch einen Benutzer. Ein Gerät gemäß Patentanspruch 3 umfasst insbesondere mindestens einen Prozessor. Der mindestens eine Prozessor ist eingerichtet um ein Verfahren durchzuführen. Das Verfahren umfasst das Erhalten einer Zeitserie von Bilddaten. Die Zeitserie von Bilddaten bildet eine Vielzahl von neurophysikalischen Ereignissen im Gehirn einer Untersuchungsperson mit überlagertem Rauschen ortsaufgelöst ab. Das Verfahren umfasst weiterhin das Durchführen einer ICA zur Identifikation einer Vielzahl von Kandidaten-Korrelationsmustern basierend auf der Zeitserie von Bilddaten. Die Vielzahl von Kandidaten-Korrelationsmustern umfasst dabei Korrelationsmuster der Vielzahl von neurophysikalischen Ereignisse sowie Falschmuster, z.B. aufgrund des Rauschen oder durch andere Effekte verursachte Intensitätsänderungen wie beispielsweise physiologische Effekte oder Erwärmung des Scanners. Das Verfahren umfasst weiterhin das Bestimmen eines Teilbereichs des Gehirns. Das Verfahren umfasst weiterhin für jedes Kandidaten-Korrelationsmuster aus der Vielzahl von Kandidaten-Korrelationsmustern: Bestimmen eines entsprechenden Werts einer vorgegebenen Metrik. Die Metrik indiziert eine Intensität des jeweiligen Kandidaten-Korrelationsmusters im Teilbereich. Das Verfahren umfasst weiterhin das Auswählen mindestens eines Kandidaten-Korrelationsmusters aus der Vielzahl von Kandidaten-Korrelationsmuster basierend auf den bestimmten Werten der vorgegebenen Metrik. Das Verfahren umfasst auch das Markieren des ausgewählten mindestens einen Kandidaten-Korrelationsmusters für eine Analyse durch einen Benutzer.

Ein Verfahren zur MRI umfasst das Erhalten einer Zeitserie von Bilddaten. Die Zeitserie bildet eine Vielzahl von neurophysikalischen Ereignissen im Gehirn einer Untersuchungsperson ortsaufgelöst ab. Das Verfahren umfasst ferner, basierend auf der Zeitserie von Bilddaten, das Durchführen einer ICA zur Identifikation einer Vielzahl von Kandidaten-Korrelationsmustern. Die Vielzahl von Kandidaten-Korrelationsmustern umfasst Korrelationsmuster der Vielzahl von neurophysikalischen Ereignissen, sowie Falschmuster. Das Verfahren umfasst ferner das Anwenden eines Computer-implementierten Klassifikators auf die Vielzahl von Kandidaten-Korrelationsmuster. Der Klassifikator ist zur Trennung der Falschmuster und der Korrelationsmuster der Vielzahl von neurophysikalischen Ereignissen eingerichtet.

Ein Computerprogrammprodukt umfasst Programmcode, der von mindestens einem Prozessor ausgeführt werden kann. Das Ausführen des Programmcodes bewirkt, dass der mindestens eine Prozessor ein Verfahren durchführt. Das Verfahren umfasst das Erhalten einer Zeitserie von Bilddaten. Die Zeitserie bildet eine Vielzahl von neurophysikalischen Ereignissen im Gehirn einer Untersuchungsperson ortsaufgelöst ab. Das Verfahren umfasst ferner, basierend auf der Zeitserie von Bilddaten, das Durchführen einer ICA zur Identifikation einer Vielzahl von Kandidaten-Korrelationsmustern. Die Vielzahl von Kandidaten-Korrelationsmustern umfasst Korrelationsmuster der Vielzahl von neurophysikalischen Ereignissen, sowie Falschmuster. Das Verfahren umfasst ferner das Anwenden eines Computer-implementierten Klassifikators auf die Vielzahl von Kandidaten-Korrelationsmuster. Der Klassifikator ist zur Trennung der Falschmuster und der Korrelationsmuster der Vielzahl von neurophysikalischen Ereignissen eingerichtet.

Ein Computerprogramm umfasst Programmcode, der von mindestens einem Prozessor ausgeführt werden kann. Das Ausführen des Programmcodes bewirkt, dass der mindestens eine Prozessor ein Verfahren durchführt. Das Verfahren umfasst das Erhalten einer Zeitserie von Bilddaten. Die Zeitserie bildet eine Vielzahl von neurophysikalischen Ereignissen im Gehirn einer Untersuchungsperson ortsaufgelöst ab. Das Verfahren umfasst ferner, basierend auf der Zeitserie von Bilddaten, das Durchführen einer ICA zur Identifikation einer Vielzahl von Kandidaten-Korrelationsmustern. Die Vielzahl von Kandidaten-Korrelationsmustern umfasst Korrelationsmuster der Vielzahl von neurophysikalischen Ereignissen, sowie Falschmuster. Das Verfahren umfasst ferner das Anwenden eines Computer-implementierten Klassifikators auf die Vielzahl von Kandidaten-Korrelationsmuster. Der Klassifikator ist zur Trennung der Falschmuster und der Korrelationsmuster der Vielzahl von neurophysikalischen Ereignissen eingerichtet.

Ein Gerät umfasst mindestens einen Prozessor, der eingerichtet ist, um ein Verfahren durchzuführen. Das Verfahren umfasst das Erhalten einer Zeitserie von Bilddaten. Die Zeitserie bildet eine Vielzahl von neurophysikalischen Ereignissen im Gehirn einer Untersuchungsperson ortsaufgelöst ab. Das Verfahren umfasst ferner, basierend auf der Zeitserie von Bilddaten, das Durchführen einer ICA zur Identifikation einer Vielzahl von Kandidaten-Korrelationsmustern. Die Vielzahl von Kandidaten-Korrelationsmustern umfasst Korrelationsmuster der Vielzahl von neurophysikalischen Ereignissen, sowie Falschmuster. Das Verfahren umfasst ferner das Anwenden eines Computer-implementierten Klassifikators auf die Vielzahl von Kandidaten-Korrelationsmuster. Der Klassifikator ist zur Trennung der Falschmuster und der Korrelationsmuster der Vielzahl von neurophysikalischen Ereignissen eingerichtet.

Eine Verwendung eines KNNs zur Identifikation von Korrelationsmustern von neurophysikalischen Ereignissen eines neuronalen Netzwerks in einer Vielzahl von Kandidaten-Korrelationsmustern ist offenbart.

Die oben dargelegten Merkmale und Merkmale, die nachfolgend beschrieben werden, können nicht nur in entsprechenden explizit dargelegten Kombinationen verwendet werden, sondern auch in weiteren Kombinationen oder isoliert, ohne den Schutzumfang der vorliegenden Erfindung zu verlassen.

### Beschreibung der Figuren:

- Figur 1: illustriert schematisch eine MR-Anlage gemäß verschiedener Beispiele, die für die erfindungsgemäßen Techniken der rsfMRI eingesetzt werden kann.
- Figur 2: ist ein Flussdiagramm eines beispielhaften Verfahrens.
- Figur 3: illustriert eine Zeitserie von Bilddaten, die gemäß verschiedener Beispiele mittels einer MR-Bildsequenz mit BOLD-Kontrast erhalten werden können.
- Figur 4: illustriert schematisch einen Zeitverlauf des Kontrasts in unterschiedlichen Voxeln der Bilddaten gemäß verschiedener Beispiele.
- Figur 5: illustriert schematisch unterschiedliche Kandidaten-Korrelationsmuster, die Komponenten einer ICA, die basierend auf den Bilddaten des Gehirns durchgeführt wird, gemäß verschiedener Beispiele entsprechen.
- Figur 6: ist ein Flussdiagramm eines beispielhaften Verfahrens.
- Figur 7: illustriert schematisch einen Teilbereich, der gemäß verschiedener Beispiele in Bezug auf das Gehirn definiert ist.
- Figur 8: illustriert schematisch ein Flussdiagramm gemäß verschiedener Beispiele.

Die oben beschriebenen Eigenschaften und Merkmale, sowie Vorteile dieser Erfindung und die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Die Figuren sind schematische Repräsentationen verschiedener Ausführungsformen der Erfindung. In den Figuren dargestellte Elemente sind nicht notwendigerweise maßstabsgetreu dargestellt. Vielmehr sind die verschiedenen in den Figuren dargestellten Elemente derart wiedergegeben, dass ihre Funktion und genereller Zweck dem Fachmann verständlich wird. In den Figuren dargestellte Verbindungen und Kopplungen zwischen funktionellen Einheiten und Elementen können auch als indirekte Verbindung oder Kopplung implementiert werden. Eine Verbindung oder Kopplung kann drahtgebunden oder drahtlos implementiert sein. Funktionelle Einheiten können als Hardware, Software oder eine Kombination aus Hardware und Software implementiert werden.

Nachfolgend werden Techniken beschrieben, um die funktionelle Konnektivität eines neuronalen Netzwerks wie beispielsweise des Gehirns einer Untersuchungsperson sichtbar zu machen. Dies bedeutet in anderen Worten, dass nachfolgend Techniken beschrieben werden, um neurophysikalische Ereignisse im Gehirn einer Untersuchungsperson sowie zugehörige Korrelationsmuster zu erkennen. Die hierin beschriebenen Techniken ermöglichen es, die neurophysikalischen Ereignisse und die zugehörigen Korrelationsmuster im Gehirn der Untersuchungsperson automatisiert und besonders zuverlässig zu erkennen. Insbesondere kann es mittels der hierin beschriebenen Techniken entbehrlich sein, dass eine Bedienperson, wie beispielsweise medizinisches Personal, eine Vielzahl von Kandidaten-Korrelationsmustern, die sowohl die Korrelationsmuster der Vielzahl von neurophysikalischen Ereignissen, jedoch auch Falschmuster beispielsweise aufgrund von Rauschen umfasst, manuell annotiert um die Falschmuster von den Korrelationsmustern der neurophysikalischen Ereignissen zu trennen.

In verschiedenen hierin beschriebenen Techniken wird eine Zeitserie von Bilddaten, die das neuronale Netzwerk mit einem geeigneten Kontrast dreidimensional abbilden, erhalten. Beispielsweise können die Bilddaten MR-Bilddaten mit BOLD-Kontrast sein. Beispielsweise könnte in den verschiedenen hierin beschriebenen Techniken eine entsprechende MR-Meßsequenz durchgeführt werden, welche die Zeitserie von Bilddaten erfasst. Beispielsweise wäre es möglich, dass die MR-Meßsequenz Bild-beschleunigt ist, beispielsweise SMS-Techniken oder eine Unterabtastung des Ortsfrequenzraum benutzt. Dadurch kann eine besonders hohe Zeitauflösung erhalten werden, sodass die Zeitserie von Bilddaten besonders viele Zeitschritte aufweist. Alternativ kann durch die Beschleunigung der Messsequenz auch eine erhöhte räumliche Ortsauflösung oder eine Mischung aus erhöhter räumlicher und zeitlicher Auflösung erzielt werden.

In den verschiedenen hierin beschriebenen Techniken kann dann eine ICA durchgeführt werden, basierend auf der Zeitserie von Bilddaten. Beispielsweise kann es in den verschiedenen hierin beschriebenen Techniken entbehrlich sein, einen Saatpunkt in Bezug auf einen Teilbereich des Untersuchungsbereich bzw. des neuronalen Netzwerks festzulegen, bevor die ICA durchgeführt wird. Mittels der ICA kann eine große Anzahl von Kandidaten-Korrelationsmustern erhalten wird, welche sowohl die tatsächlichen Korrelationsmuster der neurophysikalischen Ereignisse - welche die funktionelle Konnektivität des neuronalen Netzwerks abbilden - wie auch Falschmuster, beispielsweise aufgrund von Rauschen, Signaländerungen beispielsweise durch Erwärmung des Scanners, bestimmte Eigenschaften des der ICA zugrundeliegenden Algorithmus und/oder etwa auch durch physiologische Effekte wie Atmung oder Herzschlag verursachte Intensitätsänderungen umfassen. Gemäß verschiedener Techniken ist es möglich, eine gezielte Trennung der Korrelationsmuster der neurophysikalischen Ereignisse von den Falschmustern durchzuführen. Insbesondere ist es in den verschiedenen hierin beschriebenen Techniken möglich, diese Trennung vollautomatisiert oder größtenteils automatisiert durchzuführen. Beispielsweise kann es mittels der hierin beschriebenen Techniken entbehrlich sein, dass medizinisches Bedienpersonal die Menge der Kandidaten-Korrelationsmuster manuell klassifiziert.

Dazu wird in verschiedenen Beispielen ein Teilbereich des Gehirns bzw. im Allgemeinen des neuronalen Netzwerks manuell, teil-automatisiert oder voll-automatisiert bestimmt. Dann wird für jedes Kandidaten-Korrelationsmuster, das aus der ICA erhalten wird, ein eindimensionaler oder mehrdimensionaler Wert einer vorgegebenen Metrik bestimmt. Dabei kann die Metrik eine Intensität des jeweiligen Kandidaten-Korrelationsmusters im Teilbereich indizieren. Es ist dann möglich, ein oder mehrere Kandidaten-Korrelationsmuster aus der Vielzahl der Kandidaten-Korrelationsmuster basierend auf den bestimmten Werten der vorgegebenen Metrik auszuwählen. Beispielsweise könnten Schwellenwertvergleiche mit einem vorgegebenen Schwellenwert durchgeführt werden. Diejenigen Kandidaten- Korrelationsmuster, die eine besonders hohe Intensität im ausgewählten Teilbereich aufweisen, beispielsweise im Vergleich zu anderen Kandidaten-Korrelationsmustern oder im Vergleich zu einer absoluten Referenz, können dann ausgewählt werden und zur Analyse durch einen Benutzer markiert werden.

Mittels des Teilbereichs ist es möglich, den Ergebnisraum der ICA - welcher der Vielzahl von Kandidaten-Korrelationsmustern entspricht - einzuschränken auf solche Kandidaten-Korrelationsmuster, die eine signifikante Intensität im Teilbereich aufweisen. Beispielsweise kann der Teilbereich einem Untersuchungsbereich (englisch Region of Interest, ROI) entsprechen, der zur Diagnose genauer analysiert werden soll. Durch die Verringerung der Größe des Ergebnisraums der ICA können die vom Benutzer zu überprüfenden Kandidaten-Korrelationsmuster stark reduziert werden. Beispielsweise wäre es möglich, dass die Anzahl der auf Grundlage der Metrik ausgewählten Kandidaten-Korrelationsmuster gegenüber der Gesamtanzahl von Kandidaten-Korrelationsmuster nicht größer als 10% ist, optional nicht größer als 5%, weiter optional nicht größer als 1%.

In manchen Beispielen kann es durch geeignete Wahl der Metrik insbesondere auch möglich sein, dass bei dem Auswählen der Kandidaten-Korrelationsmuster basierend auf den bestimmten Werten der vorgegebenen Metrik gleichzeitig eine Trennung zwischen Falschmustern und Korrelationsmustern der neurophysikalischen Ereignissen erfolgen kann. Dies bedeutet in anderen Worten, dass es durch geeignete Wahl der Metrik auch möglich sein kann, mit einer besonders hohen Wahrscheinlichkeit lediglich Korrelationsmuster der neurophysikalischen Ereignisse auszuwählen und derart eine Trennung zwischen Korrelationsmuster der neurophysikalischen Ereignissen und Falschmustern zu erreichen.

Dabei wird in solchen Techniken im Gegensatz zu Referenzimplementierungen der Teilbereich nicht im Rahmen des Durchführens der ICA berücksichtigt. Der Teilbereich kann erst nach dem Durchführen der ICA berücksichtigt werden. Dies bedeutet, dass die Auswertung der Zeitserie von Bilddaten ohne eine eine Saatregion betreffende a-priori Modellannahme durchgeführt wird.

Ein weiteres Beispiel zur Auswertung der Vielzahl von Kandidaten-Korrelationsmustern beruht auf der Verwendung eines geeignet trainierten Computer-implementierten Klassifikators. Der Klassifikator kann eine Trennung der Falschmustern und der Korrelationsmuster der Vielzahl von neurophysikalischen Ereignissen erreichen. Beispielsweise könnte ein künstliches neuronales Netzwerk (KNN), wie etwa ein künstliches neuronales Faltungsnetzwerk (englisch Convolutional Neural Network, CNN) verwendet werden.

Zum Beispiel kann das KNN mehrere Schichten aufweisen. Zum Beispiel kann das KNN eine Eingangsschicht, mehrere versteckte Zwischenschichten und eine Ausgabeschicht umfassen. Dabei kann die Eingangsschicht eine Menge von Neuronen umfassen, wobei die Anzahl der Neuronen der Anzahl von Bildpunkten eines Kandidaten-Korrelationsmusters - bzw. einer durch die ICA identifizierten Komponente - entspricht. Dann können der Eingangsschicht als Eingangswerte (englisch input feature map) jeweils die verschiedenen Kandidaten-Korrelationsmuster übergeben werden. Die Eingangsschicht kann über mehrere versteckte Zwischenschichten (engl. hidden layers) mit der Ausgabeschicht verbunden sein. In der Ausgabeschicht kann für jede zu klassifizierende Kategorie ein entsprechendes Neuron existieren, welches als Ausgabewert die Zugehörigkeit des jeweils übergebenen Kandidaten-Korrelationsmusters zu der jeweiligen Kategorie liefert (engl. output feature map) .

Dabei können in den verschiedenen hierin beschriebenen Beispielen unterschiedliche Arten von Trennung erfolgen.

Erfindungsgemäss enthält in einer einfachen Implementierung die Ausgabeschicht zwei Neuronen, eines für die Korrelationsmuster der neurophysikalischen Ereignisse und eines für die Falschmuster. Es könnte aber auch zwischen mehr als lediglich zwei Klassen unterschieden werden. Beispielsweise könnte der Klassifikator weiterhin zur Trennung zwischen Korrelationsmustern von durch physiolgische Ereignisse hervorgerufenen neurophysikalischen Ereignisse und Korrelationsmustern von spontanen neurophysikalischen Ereignissen eingerichtet sein. Dies bedeutet, dass zwischen durch zum Beispiel Herzschlag oder Atmung hervorgerufenen neurophysikalischen Ereignissen und assoziierten Korrelationsmustern und anderen neurophysikalischen Ereignissen und assoziierten Korrelationsmustern mittels des Klassifikators unterschieden werden kann.

Es wäre weiterhin auch möglich, dass der Klassifikator weiterhin zur Trennung zwischen Korrelationsmustern von unterschiedlichen Typen von spontanen neurophysikalischen Ereignissen eingerichtet ist. Beispielsweise könnten die Korrelationsmuster der unterschiedlichen spontanen neurophysikalischen Ereignisse dadurch klassifiziert werden, welche Regionen des Gehirns eine besonders große entsprechende Intensität aufweisen.

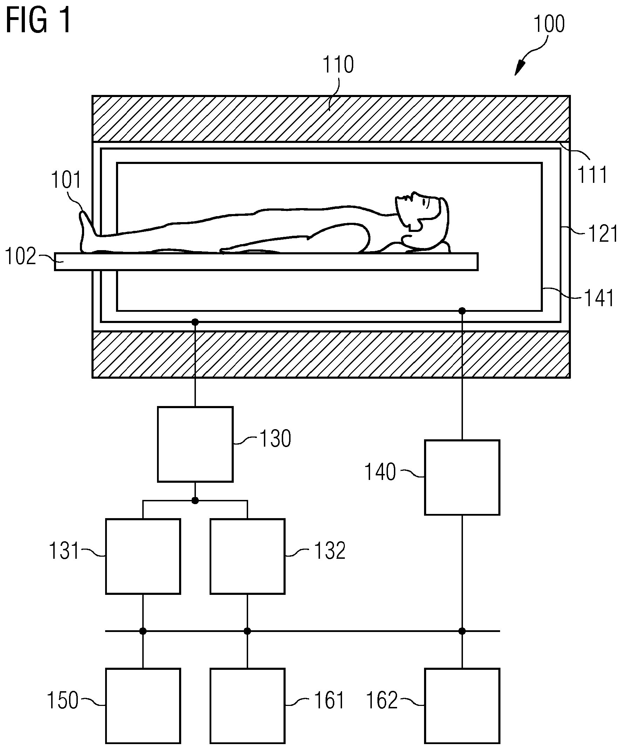

FIG. 1 illustriert schematisch eine MR-Anlage 100, die zum Durchführen der oben beschriebenen Techniken und der Techniken, die nachfolgend beschrieben werden, verwendet werden kann. Die MR-Anlage 100 weist einen Magneten 110 auf, der eine Röhre 111 definiert. Der Magnet 110 kann ein Grundmagnetfeld parallel zu seiner Längsachse erzeugen.

Ein Untersuchungsobjekt, hier eine Untersuchungsperson 101, kann auf einem Liegetisch 102 in den Magneten 110 geschoben werden. Ein Untersuchungsbereich der Untersuchungsperson kann bei der MR-Bildgebung berücksichtigt werden. Der Untersuchungsbereich ist in dem Beispiel der FIG. 1 im Bereich des Kopfes der Untersuchungsperson 101 angeordnet, insbesondere im Bereich des Gehirns. Das Gehirn bildet ein neuronales Netzwerk und neurophysikalische Ereignisse des Gehirns sollen sichtbar gemacht werden.

Die MR-Anlage 100 weist weiterhin ein Gradientensystem 140 zur Erzeugung von Gradientenfeldern auf, die für MR-Bildgebung und zur Ortskodierung von erfassten MR-Daten verwendet werden. Typischerweise umfasst das Gradientensystem 140 mindestens drei separat ansteuerbare und zueinander wohldefiniert positionierte Gradientenspulen 141. Die Gradientenspulen 141 ermöglichen es, entlang bestimmter Raumrichtungen (Gradientenachsen) Gradientenpulse anzuwenden, welche die Gradientenfelder erzeugen. Diese Gradientenachsen definieren ein Maschinenkoordinatensystem. Die Gradientenfelder können z.B. zur Schichtselektion, zur Frequenzkodierung (in Ausleserichtung) und zur Phasenkodierung verwendet werden. Dadurch kann eine Ortskodierung der MR-Daten erreicht werden.

Zur Anregung der sich im Grundmagnetfeld ergebenden Polarisation bzw. Ausrichtung der Kernmagnetisierung in Längsrichtung, ist eine HF-Spulenanordnung 121 vorgesehen, die einen amplitudenmodulierten und/oder frequenzmodulierten HF-Anregungspuls in die Untersuchungsperson 101 einstrahlen kann. Dadurch kann eine Transversalmagnetisierung erzeugt werden. Zur Erzeugung solcher HF-Anregungspulse wird ein HF-Sendesystem 131 über einen HF-Schalter 130 mit der HF-Spulenanordnung 121 verbunden. Das HF-Sendesystem 131 kann einen HF-Generator und eine HF-Amplitudenmodulationseinheit umfassen. Die HF-Anregungspulse können die Transversalmagnetisierung 1D schichtselektiv oder 2D/3D ortsselektiv oder global aus der Ruhelage kippen. Alternativ oder zusätzlich zu den HF-Anregungspulsen können auch HF-Refokussierungspulse eingestrahlt werden. Dann kann z.B. bei SMS-Messsequenzen die Transversalmagnetisierung in mehreren Schichten gleichzeitig modifiziert werden.

Weiterhin ist ein HF-Empfangssystem 132 über den HF-Schalter 130 mit der HF-Spulenanordnung 121 gekoppelt. Mit Hilfe des HF-Empfangssystems 132 können MR-Signale der relaxierenden Transversalmagnetisierung, z.B. durch induktives Einkoppeln in die HF-Spulenanordnung 121, als MR-Daten erfasst bzw. gemessen werden. Bei der SMS-Messsequenz können MR-Daten aus mehr als einer Schicht gleichzeitig erhalten werden.

Typischerweise liegen die MR-Daten zunächst als Rohdaten im Ortsfrequenzraum vor. Bei Verwendung von bildbeschleunigten MR-Messsequenzen kann eine Unterabtastung des Ortsfrequenzraum vorhanden sein. In solchen Fällen kann es erforderlich sein, die MR-Daten gegebenenfalls zu rekonstruieren bzw. zu vervollständigen und anschließend in den Bildraum zum Erhalten von Bilddaten zu transformieren. Eine Recheneinheit 161 der MR-Anlage 100 kann entsprechend eingerichtet sein.

Die MR-Anlage 100 weist weiterhin eine Bedieneinheit 150 auf, welche z.B. einen Bildschirm, eine Tastatur, eine Maus etc. umfassen kann. Mittels der Bedieneinheit 150 kann Benutzereingabe erfasst werden und Ausgabe zum Benutzer realisiert werden. Zum Beispiel kann es möglich sein, mittels der Bedieneinheit 150 einzelne Betriebsmodi bzw. Betriebsparameter der MR-Anlage 100 durch den Benutzer und/oder automatisch und/oder ferngesteuert einzustellen.

Die MR-Anlage 100 weist auch die Recheneinheit 161 auf. Beispielsweise könnte die Recheneinheit 161 ein Prozessor, ein Mikroprozessor oder ein Mikrocontroller sein. Es wäre auch möglich, dass die Recheneinheit 161 ein Feld-programmiertes Array (FPGA) oder ein applikationsspezifischer integrierter Schaltkreis (ASIC) ist. Die MR-Anlage 100 weist ferner einen Speicher 162 auf. Beispielsweise wäre es möglich, dass Steueranweisungen in dem Speicher 162 gespeichert sind, wobei die Steueranweisungen von der Recheneinheit 161 ausgeführt werden können. Ausführen der Steueranweisungen durch die Recheneinheit 161 kann bewirken, dass die Recheneinheit 161 verschiedene Techniken in Bezug auf das Bestimmen einer Messsequenz auf Grundlage einer Repräsentation der Impulsantwort des Gradientensystems 140 im K-Raum gemäß verschiedener hierin beschriebenen Beispiele ausführt.

Ausführen der Steueranweisungen durch die Recheneinheit 161 kann bewirken, dass die Recheneinheit 161 verschiedene Techniken ausführt. Beispielsweise könnte die Recheneinheit 161 Techniken in Bezug auf die SMS-Bildgebung durchführen, sowie in Bezug auf die Nachverarbeitung einer Zeitserie von Bilddaten im Zusammenhang mit der rsfMRI. Beispielsweise könnte die Recheneinheit 161 Techniken in Bezug auf eine ICA zur Identifikation einer Vielzahl von Kandidaten-Korrelationsmuster, die Korrelationsmuster von neurophysikalische Ereignissen sowie Falschmuster umfassen, durchführen. Beispielsweise könnte die Recheneinheit 161 anschließend Techniken durchführen, um die Falschmuster von den Korrelationsmustern der neurophysikalischen Ereignisse aus den durch die ICA identifizierten Komponenten bzw. Kandidaten-Korrelationsmuster zu trennen. Dazu könnte die Recheneinheit 161 beispielsweise einen Computer-implementierten Klassifikator und/oder eine Metrik, die eine Intensität der verschiedenen Kandidaten-Korrelationsmuster in einem Teilbereich des Gehirns indiziert, anwenden.

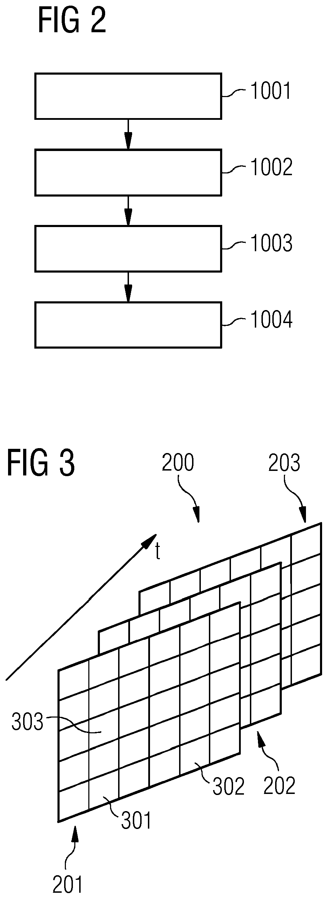

Figur 2 ist ein Flussdiagramm eines beispielhaften Verfahrens. Beispielsweise könnte das Verfahren gemäß Figur 2 von der Recheneinheit 161 durchgeführt werden.

Zunächst wird in 1001 eine Zeitserie von Bilddaten erhalten. Beispielsweise könnte in 1001 eine MR-Meßsequenz durchgeführt werden, um Rohdaten mit BOLD-Kontrast zu erhalten. Beispielsweise könnte die MR-Meßsequenz eine SMS-Bildgebung mit Unterabtastung des Ortsfrequenzraums, sowie gleichzeitiger Anregung und Auslesung von mehreren Schichten umfassen. Beispielsweise könnte in 1001 eine Rekonstruktion von unterabgetasteten Rohdaten erfolgen, beispielsweise mit Techniken der teilweisen parallelen Akquisition wie Grappa Griswold, Mark A., et al. "Generalized autocalibrating partially parallel acquisitions (GRAPPA)." Magnetic resonance in medicine 47.6 (2002): 1202-1210.

In 1002 können verschiedene Schritte der Vorverarbeitung erfolgen. 1002 ist optional.

Beispielsweise wäre es möglich, dass im Rahmen der Vorverarbeitung ein Ausgleich einer Bewegung der Untersuchungsperson erfolgt. Beispielsweise könnte ein Ausgleich im sub-Voxelbereich erfolgen. Zum Beispiel könnte eine rigide Bewegungskorrektur durchgeführt werden. Siehe zum Beispiel Thesen, Stefan, et al. "Prospective acquisition correction for head motion with image-based tracking for real-time fMRI." Magnetic Resonance in Medicine 44.3 (2000): 457-465.

Ein weiterer Aspekt einer entsprechenden Vorverarbeitung kann beispielsweise eine Normalisierung des Kontrasts in den Bilddaten betreffen. Derart können zeitliche Drifts der Amplitude des Kontrasts kompensiert werden.

Keine weitere Technik, die im Zusammenhang mit der Vorverarbeitung der Bilddaten berücksichtigt werden kann, betrifft eine Filterung des zeitlichen Verlaufs des Kontrasts, beispielsweise für individuelle Voxel der Bilddaten. Beispielsweise könnte eine zeitliche Bandpass-Filterung durchgeführt werden. Typischerweise liegt im Zusammenhang mit der rsfMRI eine Frequenz der beobachteten Signalveränderungen in einem speziellen Frequenzband, beispielsweise im Bereich von 8 mHz bis 150 mHz. Siehe beispielsweise Greicius, Michael D., et al. "Regional analysis of hippocampal activation during memory encoding and retrieval: fMRI study." Hippocampus 13.1 (2003): 164-174.

Eine weitere Technik, die im Zusammenhang mit der vor Verarbeitung in 1001 berücksichtigt werden kann, betrifft eine räumliche Filterung. Beispielsweise könnte ein Gauß-Kernel mit einer Halbwertsbreite von 3-4 mm angewendet werden. Derart können Signalartefakte reduziert werden.

Es kann im Rahmen der Vorverarbietung auch eine zeitliche Korrektur der Bilddaten, die unterschiedliche Schichten innerhalb des Gehirns abbilden, vorgenommen werden. Beispielsweise kann der Art der Zeitversatz zwischen dem Erfassen von MR-Daten in unterschiedlichen Schichten kompensiert werden.

Die oben dargelegten Beispiele von Techniken, die im Rahmen der Vorverarbeitungen 1002 beschrieben wurden, sind beispielhafte Techniken. In anderen Beispielen wäre es möglich, nur einzelne der voranstehend beschriebenen Techniken anzuwenden oder aber andere Techniken zum Vorverarbeiten der Bilddaten in 1002 anzuwenden.

Anschließend erfolgt in 1003 das Durchführen der ICA. Auf Grundlage der ICA wird eine Vielzahl von Kandidaten-Korrelationsmuster identifiziert. Die Kandidaten-Korrelationsmuster entsprechen unabhängigen Komponenten, die durch die ICA erkannt werden. Durch Überlagerung der unabhängigen Komponenten kann die Zeitserie von Bilddaten beschrieben werden. Jedes Kandidaten-Korrelationsmuster kann beispielsweise eine bestimmte Intensität in unterschiedlichen Voxeln der Bilddaten indizieren. Jedes Kandidaten-Korrelationsmuster kann beispielsweise mit einer bestimmten Zeitabhängigkeit eines zugrunde liegenden Signalverlaufs assoziiert sein. Dabei können grundsätzlich unterschiedliche Voxel, die zu einem Kandidaten-Korrelationsmuster beitragen, gleiche, komplementäre oder anderweitig phasenverschobene Signalverläufe zueinander aufweisen.

Beispielsweise kann im Rahmen der ICA der Signalverlauf der verschiedenen Voxel der Zeitserie von Bilddaten als Linearkombination von unterschiedlichen Komponenten beschrieben werden. Jeder Zeitpunkt kann beispielsweise einer Beobachtung entsprechen. Dabei kann die Anzahl der Zeitpunkte also die Anzahl von Beobachtungen bezeichnen, die aus einer bestimmten anderen Anzahl von Komponenten erzeugt wird. In anderen Worten kann die ICA eingerichtet sein, um eine Mischung aus zugrundeliegenden Komponenten zu identifizieren, welche die gemessenen Bilddaten beschreiben.

Dabei stellt die ICA solche Komponenten bereit, die eine maximale Unabhängigkeit voneinander aufweisen. Die ICA kann auf den gesamten Messbereich für unterschiedliche Voxel aufgelöst angewendet werden. Details zur ICA im Zusammenhang mit der Ruhezustands fMRI sind zum Beispiel beschrieben in: Beckmann, Christian F., et al. "Investigations into resting-state connectivity using independent component analysis." Philosophical Transactions of the Royal Society of London B: Biological Sciences 360.1457 (2005): 1001-1013. Dabei ist die Anzahl der tatsächlichen Quellen, d.h. der Korrelationsmuster tatsächlicher neurophysikalischer Ereignisse, nicht notwendigerweise gleich der Anzahl von Kandidaten-Korrelationsmuster, die im Rahmen der ICA als Komponenten identifiziert werden.

Deshalb erfolgt in 1004 das Trennen von Falschmustern und Korrelationsmustern der neurophysikalischen Ereignissen basierend auf der von der ICA identifizierten Vielzahl von Kandidaten-Korrelationsmuster.

Figur 3 illustriert Aspekte in Bezug auf eine Zeitserie 200 von Bilddaten 201-203. In Figur 3 ist dargestellt, dass zu unterschiedlichen Zeitpunkten unterschiedliche Bilddaten 201-203 erhalten werden. In Figur 3 ist ein Beispiel dargestellt, in dem zu unterschiedlichen Zeitpunkten jeweils zweidimensionale Bilddaten 201-203 einer bestimmten Schicht eines Untersuchungsbereich erhalten werden. In anderen Beispielen wäre es aber auch möglich, dass pro Zeitpunkt jeweils dreidimensionale Bilddaten 201-203 erhalten werden, die einen dreidimensionalen Untersuchungsbereich, beispielsweise das Gehirn einer Untersuchungsperson, abbilden.

Jeder Bildpunkt der Bilddaten kann dabei einen Kontrast aufweisen, der einem in einem Voxel des Untersuchungsbereichs integriertem Signal entspricht. Der Kontrast kann beispielsweise ein BOLD-Kontrast sein.

Die Bilddaten 201-203 können beispielsweise mit einer MR-Meßsequenz erhalten werden. Die Bilddaten 201-203 können zum Beispiel geeignet vorverarbeitet werden, beispielsweise in Schritt 1002 gemäß dem Verfahren aus Figur 2.

In Figur 3 sind auch drei Bildpunkte 301-303 hervorgehoben.

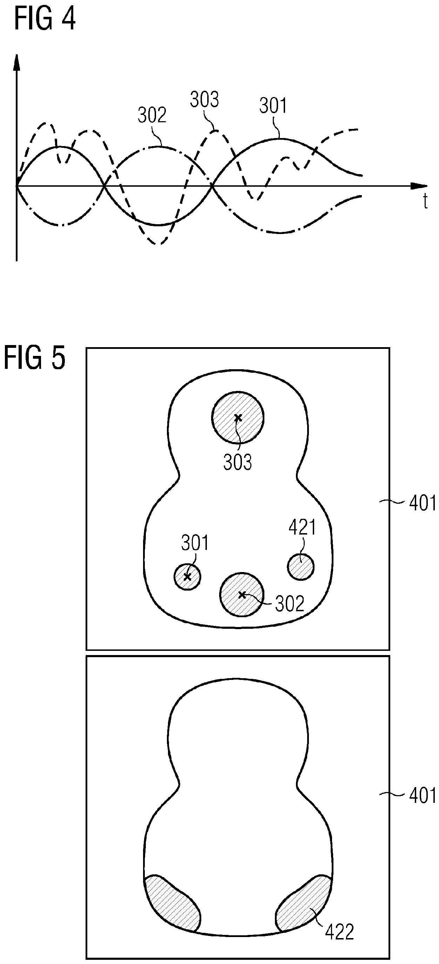

Figur 4 illustriert Aspekte in Bezug auf einen zeitlichen Signalverlauf des Kontrasts in den Bildpunkten 301-303. Aus Figur 4 ist ersichtlich, dass der Kontrast in den Bildpunkten 301-303 als Funktion der Zeit variiert. Die Anzahl der Zeitpunkte für die der Kontrast in den verschiedenen Bildpunkten verfügbar ist, wird manchmal auch als Anzahl von Beobachtungen bezeichnet. Jeder Zeitpunkt kann also eine Beobachtung darstellen. Bei der beschleunigten Bildgebung kann die Zeitauflösung und damit die Anzahl der Beobachtungen besonders groß sein.

In Figur 4 weist beispielsweise der Kontrast im Bildpunkt 301 eine große Korrelation zum Signalverlauf des Kontrasts im Bildpunkt 302 auf. Dies ist der Fall, weil der Signalverlauf im Bildpunkt 301 gegenphasig zum Signalverlauf im Bildpunkt 302 ausgebildet ist. Dahingegen weist der Signalverlauf im Bildpunkt 303 keine besonders große Korrelation mit den Signalverläufen in den Bildpunkten 301, 302 auf. Im Rahmen der ICA könnte also identifiziert werden, dass die Signalverläufe in den Bildpunkten 301, 302 zu einem gemeinsamen Kandidaten-Korrelationsmuster beitragen.

Figur 5 illustriert Aspekte in Bezug auf das Ergebnis der ICA. In Figur 5 ist ein Bild 401 dargestellt, welches eine Schicht des Gehirns abbildet. In Figur 5 ist ein Transversalschnitt durch das Gehirn dargestellt.

Dem Bild 401 überlagert sind Schwellenwert-gefilterte, ortsaufgelöste Intensitäten unterschiedlicher Kandidaten-Korrelationsmuster 421, 422, die durch die ICA identifiziert wurden, dargestellt (oben und unten in FIG. 5). Die Intensitäten können positive und negative Werte annehmen. Die Kandidaten-Korrelationsmuster 421, 422 entsprechen dabei unterschiedlichen Komponenten der ICA.

In Figur 5 sind zwei Kandidaten-Korrelationsmuster 421, 422 dargestellt, die Korrelationsmuster tatsächlicher neurophyiskalischer Ereignisse darstellen. Beispielsweise ist das Korrelationsmuster 421 das sogenannte default-mode network, siehe Van Den Heuvel, Martijn P., and Hilleke E. Hulshoff Pol. "Exploring the brain network: a review on resting-state fMRI functional connectivity." European neuropsychopharmacology 20.8 (2010): 519-534, dort Figur 2. Das Korrelationsmuster 422 ist das extra-striate visual, siehe ebd..

Die Kandidaten-Korrelationsmuster 421, 422 zeigen eine große Intensität (schraffierte Bereiche in Figur 5) in disjunkten Bereichen des Gehirns. Es ist möglich, dass Kandidaten-Korrelationsmuster identifiziert werden, die eine signifikante Intensität in überlappenden Bereichen des Gehirns aufweisen (in Figur 5 nicht dargestellt). Ferner ist in Figur 5 ein Szenario dargestellt, bei dem lediglich zwei Kandidaten-Korrelationsmuster 421, 422 identifiziert werden. Im Allgemeinen ist es jedoch möglich, dass eine wesentlich größere Anzahl von Kandidaten-Korrelationsmuster identifiziert wird. Insbesondere ist es möglich, dass Kandidaten-Korrelationsmuster identifiziert werden, die nicht Korrelationsmustern der neurophysikalischen Ereignissen entsprechen, sondern vielmehr Falschmustern, beispielsweise aufgrund von Rauschen (in Figur 5 nicht dargestellt). Nachfolgend werden Techniken beschrieben, die es ermöglichen, Korrelationsmuster der neurophysikalische Ereignisse von Falschmustern zu trennen. Beispielsweise können solche Techniken im Rahmen von 1004 in Figur 2 durchgeführt werden.

Solche Techniken können insbesondere in Anbetracht der Tatsache durchgeführt werden, dass die ICA die Kandidaten-Korrelationsmuster nicht in einer vorbestimmten oder deterministischen Reihenfolge bereit stellt. Außerdem kann das Vorzeichen der Korrelationsmuster nicht deterministisch varrieren. Darüber hinaus kann die Anzahl der Kandidaten-Korrelationsmuster abhängigen von der Anzahl der Beobachtungen. Deshalb kann bei einer besonders großen Anzahl von Beobachtungen eine große Anzahl von Falschereignissen erhalten werden. Solche Randbedingungen des der ICA zugrundeliegenden Algorithmus können in den verschiedenen hierin beschriebenen Beispielen berücksichtigt werden.

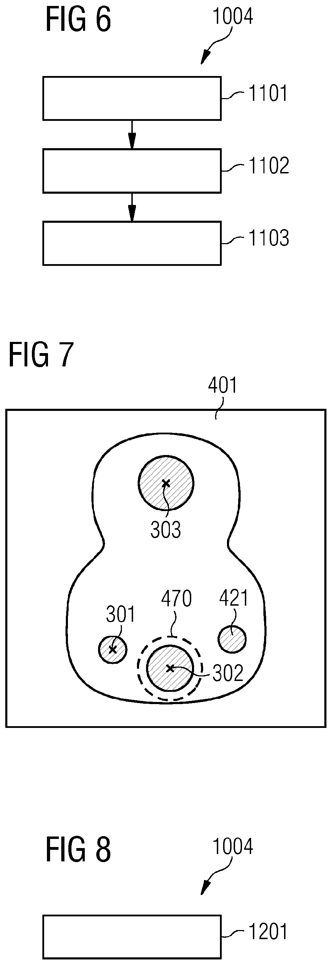

Figur 6 ist ein Flussdiagramm eines beispielhaften Verfahrens. Beispielsweise kann das Verfahren gemäß Figur 6 zum Trennen von Korrelationsmustern der neurophysikalischen Ereignissen und Falschmustern auf Grundlage einer Vielzahl von Kandidaten-Korrelationsmuster, die durch eine ICA identifiziert wurden, verwendet werden. Das Verfahren gemäß Figur 6 kann beispielsweise in 1004 aus Figur 2 durchgeführt werden.

Zunächst erfolgt in 1101 das Bestimmen eines Teilbereichs des Gehirns (vergleiche Figur 7 wo ein Teilbereich 470 des Gehirns in dem Bild 401 mit der gestrichelten Linie hervorgehoben ist). In manchen Beispielen wäre es möglich, dass der Teilbereich 470 des Gehirns manuell durch einen Benutzer bestimmt wird. In anderen Beispielen wäre es auch möglich, dass anatomische Regionen des Gehirns basierend auf den Bilddaten 201-203 oder Referenzbilddaten, die beispielsweise durch eine Referenz-MR-Meßsequenz erhalten werden, segmentiert werden. Dann kann der Teilbereich basierend auf dem Segmentierten bestimmt werden. Zum Beispiel könnte das Segmentierten auch eine Registrierung zwischen den Bilddaten und Referenzbilddaten, bei denen die anatomischen Regionen annotiert sind, umfassen. Beispielsweise kann in anderen Worten in 1101 eine automatisierte Bestimmung des Teilbereichs erfolgen. Dies kann beispielsweise auf eine Registrierung eines anatomischen Atlasses erfolgen. Dabei kann zum Beispiel der Benutzer aus einer Liste bekannter Hirnregionen die für ihn relevante Region als Teilbereich 470 auswählen.

Anschließend erfolgt in 1102 das Bestimmen eines z.B. ein- oder multidimensionalen Werts einer vorgegebenen Metrik für jedes Kandidaten-Korrelationsmuster, dass durch die ICA zuvor identifiziert wurde. Dabei beschreibt die Metrik eine Intensität des jeweiligen Kandidaten-Korrelationsmusters im Teilbereich.

Anschließend erfolgt in 1103 das Auswählen von mindestens einem Kandidaten-Korrelationsmuster auf Grundlage der bestimmten Werte der vorgegebenen Metrik für die verschiedenen Kandidaten-Korrelationsmuster. Beispielsweise könnte den 1103 solche Kandidaten-Korrelationsmuster ausgewählt werden, die in den Teilbereich eine besonders große oder eine besonders geringe Intensität aufweisen. Dazu kann beispielsweise ein Schwellenwertvergleich durchgeführt werden. Ausgewählte Kandidaten-Korrelationsmuster können dann für eine Analyse durch einen Benutzer markiert werden. Beispielsweise wäre es möglich, ausgewählte Kandidaten-Korrelationsmuster über die Benutzerschnittstelle auszugeben. Zum Beispiel könnte der Benutzer eine sortierte Liste von Kandidaten-Korrelationsmuster erhalten, wobei die Liste gemäß den jeweils bestimmten Werten der vorgegebenen Metrik sortiert ist.

Durch solche Techniken kann eine Reduktion des Ergebnisraums, der vom Benutzer analysiert werden muss, erzielt werden. Insbesondere kann eine Filterung in Bezug auf solche Kandidaten-Korrelationsmuster erzielt werden, die in Bezug auf den Teilbereich 470 - z.B. eine ROI - relevant sind.

Dabei sollte beachtet werden, dass die ICA in einem Bereich des Gehirns, der den Teilbereich einschließt und größer als der Teilbereich 470 ist, oder im ganzen Gehirn durchgeführt wird. In anderen Worten beeinflusst die Wahl des Teilbereichs nicht das Durchführen der ICA. Im Gegensatz zu Referenzimplementierungen wird der ausgewählte Teilbereich 470 also nicht als Grundlage zur Identifikation der Kandidaten-Korrelationsmuster herangezogen. Stattdessen wird der Teilbereich 470 erst im Anschluss an die ICA verwendet, nämlich um eine Trennung von Falschmustern von Korrelationsmustern der neurophysikalischen Ereignisse zu unterstützen.

Dabei kann eine geeignete Wahl der Metrik eine genaue Trennung zwischen Korrelationsmustern der neurophysikalischen Ereignissen und Falschmustern unterstützen. Dies kann durch Berücksichtigung der Eigenschaften der durch die ICA extrahierten Komponenten bei der Wahl der Metrik gefördert werden. Beispielsweise beruhen manche Techniken der Wahl der Metrik auf der Erkenntnis, dass die ICA die Kandidaten-Korrelationsmuster in einer nicht deterministischen Reihenfolge und auch mit variabler Varianz und variablem Vorzeichen bestimmt. Deshalb kann es in manchen Beispielen möglich sein, dass die Metrik eine normalisierte Varianz einer Komponente der Zeitserie von Bilddaten, die dem jeweiligen Kandidaten-Korrelationsmuster entspricht, berücksichtigt.

Die Metrik kann einen über den Teilbereich 470 integrierte Intensität einer Komponente der Zeitserie 200 von Bilddaten 201-203, die dem jeweiligen Kandidaten-Korrelationsmuster entspricht, berücksichtigen. Beispielsweise könnte also die Metrik eine integrierte Intensität der normalisierten Varianz einer Komponente als Integral über den Teilbereich 470 berücksichtigen. Dabei kann der Teilbereich 470 zum Beispiel zweidimensional oder dreidimensional definiert sein; und das Integral kann entsprechend zweidimensional oder dreidimensional definiert sein.

Es ist auch möglich, dass die Metrik einen Betrag der Intensität einer Komponente der Zeitserie 200 von Bilddaten 201-203, die dem jeweiligen Kandidaten-Korrelationsmuster entspricht, berücksichtigt. Dies bedeutet, dass das Vorzeichen der Intensität eines jeweiligen Kandidaten-Korrelationsmusters unberücksichtigt verbleiben kann. Derart können beliebige Vorzeichen, die durch die ICA für die unterschiedlichen Komponenten erhalten werden, bei der Ermittlung der Intensität des entsprechenden Kandidaten-Korrelationsmusters in den Teilbereich 170 kompensiert werden. Dies beruht auf der Erkenntnis, dass das Vorzeichen der Intensität der verschiedenen Kandidaten-Korrelationsmuster oftmals ohne physikalisch relevanten Inhalt ist.

In manchen Beispielen könnte die Metrik eine Differenz zwischen Intensitäten einer Komponente der Zeitserie 200 von Bilddaten 201-203, die dem jeweiligen Kandidaten-Korrelationsmuster entspricht, innerhalb und außerhalb des Teilbereichs 470 berücksichtigen. Derart könnten beispielsweise solche Kandidaten-Korrelationsmuster erkannt werden, die eine besonders gleichförmige Intensität innerhalb und außerhalb des Teilbereichs 470 aufweisen. Solche Kandidaten-Korrelationsmuster können zum Beispiel als Falschmuster identifiziert werden, weil sie zum Beispiel aufgrund von Rauschen hervorgerufen werden und gleichförmig im Bereich des Gehirns bzw. über ausgedehnte Bereiche des Gehirns und nicht beschränkt auf einen anatomisch relevanten Teilbereich 470 aktiviert sind. Derart kann also eine besonders effiziente Trennung zwischen Falschmustern und Korrelationsmustern der neurophysikalischen Ereignissen erfolgen.

Solche Techniken, wie sie in Bezug auf das Verfahren gemäß Figur 6 beschrieben wurden, ermöglichen also eine automatisierte Erkennung von Korrelationsmustern der neurophysikalischen Ereignissen auf Grundlage einer Vielzahl von Kandidaten-Korrelationsmuster, die auch Falschmuster umfassen. Insbesondere kann eine solche Erkennung ohne fehleranfällige und zeitaufwendige manuelle Definition von Saatregionen als a-priori Einschränkung erfolgen. Ein Teilbereich kann im Anschluss an das Durchführen der ICA berücksichtigt werden. Dabei kann die Konsistenz der erkannten Korrelationsmustern der neurophysikalischen Ereignisse im Vergleich zu manuell definierten Saatregionen als a-priori Einschränkung höher sein, da die ICA beispielsweise keine inkonsistenten "Mischregionen" erzeugt. So ist es beispielsweise in Referenztechniken, die auf einem Saatpunkt als a-priori Einschränkung basieren, möglich, eine Saatregion zu definieren, die anatomisch und aus Gesichtspunkten der Konnektivität keine einzelne abgeschlossene Region bildet. Darunter leidet die Qualität der extrahierten Komponenten erheblich. Darüber hinaus ist die Lösung dieses Problems nicht trivial, da zum Zeitpunkt der Bestimmung der Saatregionen das Ergebnis der ICA noch nicht zur Verfügung steht. Solche Nachteile werden durch die hierin beschriebenen Techniken vermieden.

In den hierin beschriebenen Techniken ist es möglich, die Kandidaten-Korrelationsmuster vollautomatisch mittels der ICA zu bestimmen. In einem davon unabhängigen Schritt ist es möglich, dass ein Teilbereich durch den Anwender spezifiziert wird, der für die aktuelle Analyse besonders relevant ist. Erst anschließend werden diese unabhängigen Informationen miteinander verknüpft, sodass ein unpräzise definierter Saatbereich als a-priori Information für die ICA die Qualität der Ergebnisse nicht oder nicht signifikant beeinflusst.

Figur 8 ist ein Flussdiagramm eines beispielhaften Verfahrens. Insbesondere illustriert Figur 8 Techniken in Bezug auf das Trennen von Korrelationsmustern der neurophysikalischen Ereignisse von Falschmustern. Beispielsweise könnte das Verfahren gemäß Figur 8 im Rahmen von 1004 in Figur 2 durchgeführt werden.

In 1201 wird ein Computer-implementierter Klassifikator auf die Vielzahl von Kandidaten-Korrelationsmuster, die im Rahmen der ICA identifiziert wurden, angewendet. Der Klassifikator ist eingerichtet, um eine Trennung der Falschmuster und der Korrelationsmuster der Vielzahl von neurophysikalischen Ereignisse zu erreichen.

Solche Effekte beruhen auf der Erkentnis, dass oftmals Falschmuster ein charakteristisches Ortsraumprofil der Intensität aufweisen können. Z.B. können Falschmuster aufgrund von Bewegung - beispielsweise bewirkt durch physiologische Effekte wie Atmung - oder aufgrund von Blutfluss - beispielsweise bewirkt durch physiologische Effekte wie Herzschlag - ein charakteristisches Ortsraumprofil aufweisen. Beispielsweise kann das Ortsraumprofil von Falschmustern aufgrund von Bewegung eine besonders hohe Intensität am Umfang des Gehirns aufweisen, weil sich entgegengesetzte Seiten des Gehirns korreliert bewegen. Aufgrund dieser Erkenntnis kann es besonders gut möglich sein, Falschmuster mittels des Computer-implementierten Klassifikators zu erkennen.

Beispielsweise könnte als Klassifikator ein KNN verwendet werden. Ein beispielhaftes KNN ist ein CNN. Entsprechende Techniken sind dem Fachmann grundsätzlich bekannt aus: Krizhevsky, Alex, Ilya Sutskever, and Geoffrey E. Hinton. "Imagenet classification with deep convolutional neural networks." Advances in neural information processing systems. 2012 und Lawrence, Steve, et al. "Face recognition: A convolutional neural-network approach." IEEE transactions on neural networks 8.1 (1997): 98-113. Bei einem CNN werden räumlich begrenzte dreidimensionale Bereiche mit dreidimensionalen Kerneln gefaltet. Dies bedeutet, dass keine volle Verknüpfung eines Neurons einer Schicht mit allen Neuronen einer vorangehenden Schicht besteht. Eine Translationsinvarianz wird dadurch erreicht, dass jeder Kernel mit allen möglichen sensitiven Bereichen gefaltet wird. Auch das CNN kann mehrere versteckte Ebenen unterstützen. Je nach Anzahl der verwendeten Ebenen können eine unterschiedliche Anzahl von Merkmalen in den Eingangsdaten, d.h. den durch die ICA bereitgestellten Bilddaten, die die Kandidaten-Korrelationsmuster abbilden, erkannt werden. Gleichzeitig wird die Anzahl der Parameter reduziert. Es können auch Pooling-Schichten verwendet werden, welche die Ergebnisse von vorangegangenen Schichten konsolidieren. Es können auch vollständig verbundene Schichten verwendet werden, typischerweise nahe bei der Ausgabeschicht.

Das Training eines KNNs kann auf Grundlage grundsätzlich bekannter Techniken erfolgen. Beispielsweise kann der Rückwärts-Propagationsalgorithmus verwendet werden, siehe Rumelhart, David E., Geoffrey E. Hinton, and Ronald J. Williams. "Learning representations by back-propagating errors." Cognitive modeling 5.3 (1988): 1. Hierfür wird typischerweise eine Menge bekannter Trainingspaare aus Komponenten einer ICA gegenüber Kategorien benötigt. Die Kategorien werden durch einen erfahrenen Benutzer beispielsweise manuell zugeordnet. Das Ergebnis des Trainings liegt dann in einer Menge an Kantengewichte für das entsprechende KNN vor, welche die jeweilige Gewichtung der Daten beim Durchgang durch das KNN von der Eingabeschicht bis hin zur Ausgabeschicht charakterisieren. Nachdem das KNN trainiert ist, ist typischerweise keine weitere Interaktion zur Klassifikation der Komponenten der ICA bzw. der Kandidaten-Korrelationsmuster erforderlich. Die Menge der Komponenten der ICA kann dann iterativ durch das KNN klassifiziert werden.

Dabei sind in unterschiedlichen Beispielen unterschiedliche Kategorien, die durch den Klassifikator erfasst werden, denkbar. Beispielsweise können die unterschiedlichen Kategorien durch geeignetes Training des Klassifikators erlernt werden. Der Klassifikator könnte etwa zur Trennung zwischen Korrelationsmustern unterschiedlicher Typen von spontanen neurophysikalischen Ereignissen eingerichtet sein. Beispiele für Korrelationsmuster spontaner neurophysikalische Ereignisse umfassen zum Beispiel "Default Mode Network", "Attention Network", etc. Die Korrelationsmuster unterschiedlicher Typen von spontanen neurophysikalischen Ereignissen sind beispielsweise beschrieben in: Greicius, Michael D., et al. "Functional connectivity in the resting brain: a network analysis of the default mode hypothesis." Proceedings of the National Academy of Sciences 100.1 (2003): 253-258.

Mittels der hierin beschriebenen Techniken ist es also möglich, eine automatisierte Auswahl relevanter Korrelationsmuster von neurophysikalischen Ereignissen auf Grundlage einer Computer-implementierten Klassifikation von Komponenten einer ICA, die Kandidaten-Korrelationsmuster entsprechen, durchzuführen. Eine manuelle Auswahl entfällt. Die Benutzerinteraktion im Zusammenhang mit der rsfMRI wird dadurch deutlich vereinfacht.

Die verschiedenen hierin beschriebenen Techniken können z.B. durch Recheneinheit 161 der MR-Anlage 100 durchgeführt werden. Es wäre aber auch möglich, Cloud-Computing einzusetzen. z.B. könnten die Kandidaten-Korrelationsmuster an einen Server übertragen werden und der Server könnte dann zur Trennung zwischen den Korrelationsmustern der neurophysikalischen Ereignisse und Falschmustern eingerichtet sein. Selbstverständlich können die Merkmale der vorab beschriebenen Ausführungsformen und Aspekte der Erfindung miteinander kombiniert werden. Insbesondere können die Merkmale nicht nur in den beschriebenen Kombinationen, sondern auch in anderen Kombinationen oder für sich genommen verwendet werden, sofern dadurch der Schutzumfang der Erfindung nicht verlassen wird.

Beispielsweise wurden voranstehend verschiedene Beispiele beschrieben, bei denen die Bilddaten aus der MRI erhalten werden. Dies ist beispielhaft und in anderen Beispielen könnten auch andere Bildgebungstechniken angewendet werden.

## Patentansprüche

1. Verfahren zur Magnetresonanz-Bildgebung, mit den folgenden Schritten
- Erhalten einer Zeitserie von Bilddaten, welche eine Vielzahl von neurophysikalischen Ereignissen im Gehirn einer Untersuchungsperson ortsaufgelöst abbildet,
- Durchführen einer Unabhängigkeitsanalyse zur Identifikation einer Vielzahl von Kandidaten-Korrelationsmustern, wobei die Vielzahl von Kandidaten-Korrelationsmustern Korrelationsmuster der Vielzahl von neurophysikalischen Ereignisse sowie Falschmuster umfasst, und
- Anwenden eines computer-implementierten Klassifikators auf die Vielzahl von Kandidaten-Korrelationsmuster, der zur Trennung der Falschmuster und der Korrelationsmuster der Vielzahl von neurophysikalischen Ereignissen eingerichtet ist, wobei der Klassifikator ein eine Eingangsschicht, mehrere versteckte Zwischenschichten und eine Ausgabeschicht umfassendes künstliches neuronales Netzwerk umfasst, beispielsweise ein künstliches neuronales Faltungsnetzwerk, und wobei die Ausgabeschicht zumindest zwei Neuronen umfasst, eines für die Korrelationsmuster der neurophysikalischen Ereignisse und eines für die Falschmuster.

2. Verfahren nach Anspruch 1,
wobei der Klassifikator weiterhin zur Trennung zwischen Korrelationsmustern unterschiedlicher Typen von spontanen neurophysikalischen Ereignissen eingerichtet ist.

3. Gerät mit mindestens einem Prozessor, der eingerichtet ist, um ein Verfahren mit folgenden Schritten durchzuführen:
- Erhalten einer Zeitserie von Bilddaten, welche eine Vielzahl von neurophysikalischen Ereignissen im Gehirn einer Untersuchungsperson ortsaufgelöst abbildet,
- Durchführen einer Unabhängigkeitsanalyse zur Identifikation einer Vielzahl von Kandidaten-Korrelationsmustern, wobei die Vielzahl von Kandidaten-Korrelationsmustern Korrelationsmuster der Vielzahl von neurophysikalischen Ereignisse sowie Falschmuster umfasst, und
- Anwenden eines computer-implementierten Klassifikators auf die Vielzahl von Kandidaten-Korrelationsmuster, der zur Trennung der Falschmuster und der Korrelationsmuster der Vielzahl von neurophysikalischen Ereignissen eingerichtet ist, wobei der Klassifikator ein eine Eingangsschicht, mehrere versteckte Zwischenschichten und eine Ausgabeschicht umfassendes künstliches neuronales Netzwerk, beispielsweise ein künstliches neuronales Faltungsnetzwerk, umfasst, und wobei die Ausgabeschicht zumindest zwei Neuronen umfasst, eines für die Korrelationsmuster der neurophysikalischen Ereignisse und eines für die Falschmuster.

## Claims

1. Method for magnetic-resonance imaging, with the following steps
- receiving a time series of image data, which depicts a plurality of neurophysical events in the brain of a person under examination in a spatially-resolved manner,
- performance of an independent component analysis to identify a plurality of candidate correlation patterns, wherein the plurality of candidate correlation patterns includes correlation patterns of the plurality of neurophysical events and false patterns, and
- application of a computer-implemented classifier to the plurality of candidate correlation patterns, which is configured to differentiate between the false patterns and the correlation patterns of the plurality of neurophysical events, wherein the classifier comprises an artificial neural network comprising an input layer, several hidden inter-layers and an output layer, for example an artificial convolutional neural network, and wherein the output layer comprises at least two neurons, one for the correlation patterns of the neurophysical events and one for the false patterns.

2. Method according to claim 1,
wherein the classifier is additionally configured to differentiate between correlation patterns of different types of spontaneous neurophysical events.

3. Device with at least one processor, which is configured to carry out a method with the following steps:
- receiving a time series of image data, which depicts a plurality of neurophysical events in the brain of a person under examination in a spatially-resolved manner,
- performance of an independent component analysis to identify a plurality of candidate correlation patterns, wherein the plurality of candidate correlation patterns includes correlation patterns of the plurality of neurophysical events and false patterns, and
- application of a computer-implemented classifier to the plurality of candidate correlation patterns, which is configured to differentiate between the false patterns and the correlation patterns of the plurality of neurophysical events, wherein the classifier comprises an artificial neural network comprising an input layer, several hidden inter-layers and an output layer, for example an artificial convolutional neural network, and wherein the output layer comprises at least two neurons, one for the correlation patterns of the neurophysical events and one for the false patterns.

## Revendications

1. Procédé d'imagerie par résonnance magnétique, comprenant les stades suivants
- on obtient une série temporelle de données d'image, qui représente en résolution spatiale une pluralité d'événements neurophysiques dans le cerveau d'une personne à examiner,
- on effectue une analyse d'indépendance pour l'identification d'une pluralité de motifs de corrélation candidats, la pluralité de motifs de corrélation candidats comprenant des motifs de corrélation de la pluralité d'événements neurophysiques, ainsi que des motifs faux, et
- on applique un classificateur assisté par ordinateur à la pluralité de motifs de corrélation candidats, qui est conçu pour la séparation des motifs faux et des motifs de corrélation de la pluralité d'événements neurophysiques, le classificateur comprenant un réseau neuronal artificiel comprenant une couche d'entrée, plusieurs couches intermédiaires cachées et une couche de sortie, en étant par exemple un réseau neuronal convolutif artificiel, et dans lequel la couche de sortie comprend au moins deux neurones, un pour les motifs de corrélation des événements neurophysiques et un pour les motifs faux.

2. Procédé suivant la revendication 1,
dans lequel le classificateur est conçu en outre pour la séparation entre des motifs de corrélation de types différents d'événements neurophysiques spontanés.

3. Appareil comprenant au moins un processeur, qui est conçu pour effectuer un procédé ayant les stades suivants :
- on obtient une série temporelle de données d'image, qui représente en résolution spatiale une pluralité d'événements neurophysiques dans le cerveau d'une personne à examiner,
- on effectue une analyse d'indépendance pour l'identification d'une pluralité de motifs de corrélation candidats, la pluralité de motifs de corrélation candidats comprenant des motifs de corrélation de la pluralité d'événements neurophysiques, ainsi que des motifs faux, et
- on applique un classificateur assisté par ordinateur à la pluralité de motifs de corrélation candidats, qui est conçu pour la séparation des motifs faux et des motifs de corrélation de la pluralité d'événements neurophysiques, le classificateur comprenant un réseau neuronal artificiel comprenant une couche d'entrée, plusieurs couches intermédiaires cachées et une couche de sortie, en étant par exemple un réseau neuronal convolutif artificiel, et dans lequel la couche de sortie comprend au moins deux neurones, un pour les motifs de corrélation des événements neurophysiques et un pour les motifs faux.
